Europäisches Patentamt

European Patent Office

Office européen des brevets

(19)

(11) **EP 0 915 821 B1**

(12) **EUROPÄISCHE PATENTSCHRIFT**

(45) Veröffentlichungstag und Bekanntmachung des Hinweises auf die Patenterteilung:
**21.11.2001 Patentblatt 2001/47**

(51) Int Cl.$^7$: **C07C 51/215**, C07C 51/25, B01J 23/652, B01J 23/89

(21) Anmeldenummer: **97937492.3**

(22) Anmeldetag: **16.07.1997**

(86) Internationale Anmeldenummer:
**PCT/EP97/03809**

(87) Internationale Veröffentlichungsnummer:
**WO 98/05619 (12.02.1998 Gazette 1998/06)**

(54) **VERFAHREN ZUR SELEKTIVEN HERSTELLUNG VON ESSIGSÄURE UND DAFÜR GEEIGNETE KATALYSATOREN**

SELECTIVE PREPARATION PROCESS OF ACETIC ACID AND CATALYSTS THEREFOR

PROCEDE DE PRODUCTION SELECTIVE D'ACIDE ACETIQUE, ET CATALYSEUR POUVANT ETRE UTILISE A CET EFFET

(84) Benannte Vertragsstaaten:
**AT BE CH DE DK ES FR GB IT LI NL SE**

(30) Priorität: **31.07.1996 DE 19630832**

(43) Veröffentlichungstag der Anmeldung:
**19.05.1999 Patentblatt 1999/20**

(73) Patentinhaber: **Celanese Chemicals Europe GmbH**
**60439 Frankfurt am Main (DE)**

(72) Erfinder:
• **BORCHERT, Holger**
**D-67278 Bockenheim a.d. Weinstrasse (DE)**
• **DINGERDISSEN, Uwe**
**D-64342 Seeheim-Jugenheim (DE)**

(56) Entgegenhaltungen:
EP-A- 0 113 156        EP-A- 0 608 838
EP-A- 0 620 205        GB-A- 2 040 717
US-A- 4 298 531        US-A- 4 499 301

• **PATENT ABSTRACTS OF JAPAN vol. 095, no. 001, 28.Februar 1995 & JP 06 293695 A (SHOWA DENKO KK), 21.Oktober 1994,**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft ein Verfahren zur selektiven Herstellung von Essigsäure durch katalytische Gasphasenoxidation von Ethan oder von Ethylen und Ethan enthaltenden Gemischen in Gegenwart eines Palladium enthaltenden Katalysators.

Die oxidative Dehydrierung von Ethan zu Ethylen in der Gasphase bei Temperaturen >500°C ist beispielsweise aus US-A-4 250 346, US-A-4 524 236 und US-A-4 568 790 bekannt.

So beschreibt die US-A-4 250 346 die Verwendung einer Katalysatorzusammensetzung, die die Elemente Molybdän, X und Y im Verhältnis a:b:c enthält zur Umwandlung von Ethan in Ethylen, worin X gleich Cr, Mn, Nb, Ta, Ti, V, und/oder W ist und Y gleich Bi, Ce, Co, Cu, Fe, K, Mg, Ni, P, Pb, Sb, Si, Sn, Tl und/oder U ist und a gleich 1, b gleich 0,05 bis 1 und c gleich 0 bis 2 ist. Der Gesamtwert von c für Co, Ni und/oder Fe muß dabei weniger als 0,5 betragen.

Die Reaktion wird vorzugsweise in Anwesenheit von zugefügtem Wasser durchgeführt. Die offenbarten Katalysatoren können ebenfalls zur Oxidation von Ethan zu Essigsäure verwendet werden, wobei die Effizienz der Umwandlung zu Essigsäure bei ca. 18 %, bei einer Ethan-Umwandlung von 7,5 %, liegt.

Die vorstehend genannten Schriften beschäftigen sich hauptsächlich mit der Herstellung von Ethylen, weniger mit der gezielten Herstellung von Essigsäure.

Dagen beschreibt die EP-B-0 294 845 ein Verfahren zur selektiven Herstellung von Essigsäure aus Ethan, Ethylen oder Gemischen davon mit Sauerstoff in Gegenart eines Katalysatorgemisches, welches mindestens A.) einen calcinierten Katalysator der Formel $Mo_xV_y$ oder $Mo_xV_yZ_y$, worin Z eines oder mehrere der Metalle Li, Na, Be, Mg, Ca, Sr, Ba, Zn, Cd, Hg, Sc, Y, La, Ce, Al, Tl, Ti, Zr, Hf, Pb, Nb, Ta, As, Sb, Bi, Cr, W, U, Te, Fe, Co und Ni sein kann, und x gleich 0,5 bis 0,9 ist, y gleich 0,1 bis 0,4 ist; und z gleich 0,001 bis 1 ist und B.) einen Ethylenhydratationskatalysator und/oder Ethylenoxidationskatalysator enthält. Bei der zweiten Katalysatorkomponente B handelt es sich insbesondere um einen Molekularsiebkatalysator oder einen Palladium enthaltenden Oxidationskatalysator. Bei der Verwendung des beschriebenen Katalysatorgemisches und Einspeisung eines Gasgemisches bestehend aus Ethan, Sauerstoff, Stickstoff und Wasserdampf durch den Katalysator enthaltenden Reaktor beträgt die maximale Selektivität 27% bei einem Ethanumsatz von 7%. Die hohen Umsatzraten von Ethan werden gemäß der EP 0 294 845 nur mit dem beschriebenen Katalysatorgemisch, nicht jedoch mit einem einzigen, die Komponenten A und B enthaltenden Katalysator erreicht.

Ein weiteres Verfahren zur Herstellung eines Produktes, das Ethylen und/oder Essigsäure enthält, wird in EP-B-0 407 091 beschrieben. Hierbei werden Ethan und/oder Ethylen und ein molekularen Sauerstoff enthaltendes Gas bei erhöhter Temperatur mit einer Katalysatorzusammensetzung, die die Elemente A, X und Y enthält, in Kontakt gebracht. A ist hierbei $Mo_dRe_eW_f$, X ist Cr, Mn, Nb, Ta, Ti, V und/oder W und Y ist Bi, Ce, Co, Cu, Fe, K, Mg, Ni, P, Pb, Sb, Si, Sn, Tl und/oder U. Die maximalen Selektivitäten, die bei Verwendung des beschriebenen Katalysators bei der Oxidation von Ethan zu Essigsäure erzielt werden konnten, betragen 78%. Als weitere Nebenprodukte werden Kohlendioxid, Kohlenmonoxid und Ethylen gebildet.

Aus EP-A1-0 620 205 ist ein Verfahren zur Herstellung von Essigsäure, ausgehend von Ethylen, bekannt. Die Essigsäureherstellung erfolgt in Gegenwart von Katalysatoren, die metallisches Palladium und Heteropolysäuren, z.B. Wolfram-Vanadium-Phosphor-Heteropolysäuren, enthalten.

US-A-4,298,531 beschreibt ein Verfahren zur Herstellung von Furan aus 1,3-Butadien. Die Umsetzung erfolgt in Gegenwart einer wäßrigen Lösung, die Palladiumsalze und Heteropolysäuren enthält.

Ferner offenbart US-A-4,499,301 ein Verfahren zur Herstellung ungesättigter Carbonsäuren, z.B. von Acrylsäure, ausgehend von Olefinen mit Hilfe eines palladiumhaltigen Katalysators, der darüber hinaus noch Gold, Silber und Kupfer sowie die Promotoren $MoO_3$ und $TiO_2$, niedergeschlagen auf einem Träger, enthält.

Keine der vorstehend aufgezählten Publikationen beschreibt jedoch die Verwendung eines Katalysators, der die Elemente Palladium, Molybdän, Niob und Vanadium enthält, zur selektiven Oxidation von Ethan oder von Ethylen und Ethan enthaltenden Gemischen zu Essigsäure. Ferner beruhen viele bekannte Katalysatoren auf Heteropolysäuren und die bis jetzt im Stand der Technik erzielten Selektivitäten für die Oxidation zu Essigsäure sind noch nicht befriedigend.

Es besteht daher die Aufgabe ein Verfahren zur Verfügung zu stellen, das es erlaubt, Ethan oder Ethylen und Ethan enthaltende Gemische in einfacher Weise, gezielt und mit hoher Selektivität unter möglichst milden Reaktionsbedingungen zu Essigsäure zu oxidieren.

Überraschend wurde nun gefunden, daß es möglich ist, bei Verwendung eines Katalysators, der die Elemente Molybdän, Palladium, Vanadium und Niob und eines oder mehrere Elemente aus der Gruppe Chrom, Mangan, Tantal, Titan, Tellur und/oder Wolfram enthält, Ethan oder Ethylen und Ethan enthaltenden Gemische unter relativ milden Bedingungen, in einfacher Weise mit hoher Selektivität zu Essigsäure zu oxidieren. Die vorliegende Erfindung betrifft somit ein Verfahren zur selektiven Herstellung von Essigsäure aus einer gasförmigen Einspeisung aus Ethan oder Gemischen aus Ethan und Ethylen sowie Sauerstoff bei erhöhter Temperatur, wobei die gasförmige Einspeisung mit einem Katalysator zusammengebracht wird, der die Elemente Mo, Pd, X und Y in den Grammatomverhältnissen a:b:

c:d in Kombination mit Sauerstoff enthält.

$$Mo_aPd_bX_cY_d \qquad\qquad (I)$$

und die Symbole X und Y folgende Bedeutung haben:

X steht für eines oder mehrere Elemente ausgewählt aus der Gruppe: Cr, Mn, Nb, Ta, Ti, V, Te und/oder W, insbesondere Nb, V und W;

Y steht für eines oder mehrere Elemente ausgewählt aus der Gruppe: B, Al, Ga, In, Pt, Zn, Cd, Bi, Ce, Co, Cu, Rh, Ir, Au, Ag, Fe, Ru, Os, K, Rb, Cs, Mg, Ca, Sr, Ba, Zr, Hf, Ni, P, Pb, Sb, Si, Sn, Tl und U, insbesondere Ca, Sb und Li mit der Maßgabe, daß der Katalysator als X mindestens die Elemente V und Nb enthält und daß Mo und die Elemente für X nicht auf einer Heteropolysäure beruhen.

Die Indizes a, b, c und d stehen für die Grammatomverhältnisse der entsprechenden Elemente, wobei a = 1, b > 0, c > 0 und d = 0 bis 2 ist.
Sofern X und Y für mehrere verschiedene Elemente stehen, können die Indices c und d ebenfalls mehrere unterschiedliche Werte annehmen.

Weiterhin betrifft die vorliegende Erfindung einen Katalysator zur selektiven Herstellung von Essigsäure enthaltend die Elemente Mo, Pd, X und Y in den Grammatomverhältnissen a:b:c:d in Kombination mit Sauerstoff.

Die Grammatomverhältnisse a:b:c:d liegen vorzugsweise in folgenden Bereichen:

a = 1;
b = 0,0001 bis 0,5;
c = 0,1 bis 1,0 und
d = 0 bis 1,0.

Palladiumgehalte im Katalysator, die über der angegebenen Obergrenze liegen führen bei dem erfindungsgemäßen Verfahren zu einer Begünstigung der Kohlendioxidbildung. Ferner werden höhere Palladiumgehalte allgemein auch deshalb vermieden, da sie den Katalysator unnötig verteuern. Dagegen wird bei Palladiumgehalten unterhalb des angegebenen Grenzwertes eine Bevorzugung der Ethylenbildung beobachtet.

Vorzugsweise enthält der erfindungsgemäß verwendete Katalysator außer den Elementen Molybdän und Palladium noch Vanadium, Niob, Antimon und Kalzium in Kombination mit Sauerstoff. Die Grammatomverhältnisse a:b:c$^1$:c$^2$:d$^1$:d$^2$ der Elemente Mo:Pd:V:Nb:Sb:Ca sind vorzugsweise wie folgt:

a (Mo) =1;
b (Pd) = 0,0001 bis 0,5, insbesondere 0,0001 bis 0,05;
c$^1$ (V) = 0,1 bis 1,0;
c$^2$ (Nb) = 0,1 bis 0,5;
d$^1$ (Sb) = 0 bis 0,5;
d$^2$ (Ca) = 0 bis 0,2;

Beispiele für derartige im erfindungsgemäßen Verfahren bevorzugt eingesetzte Katalysatorzusammensetzungen sind:

$Mo_{1,00}V_{0,25}Nb_{0,12}Pd_{0,0005}$
$Mo_{1,00}V_{0,25}Nb_{0,12}Pd_{0,0004}$
$Mo_{1,00}V_{0,25}Nb_{0,12}Pd_{0,0003}$
$Mo_{1,00}V_{0,36}Nb_{0,03}Sb_{0,01}Ca_{0,01}Pd_{0,0005}$
$Mo_{1,00}V_{0,50}Nb_{0,15}Te_{0,2}Pd_{0,0002}$
$Mo_{1,00}V_{0,25}Nb_{0,3}W_{0,2}Pd_{0,0003}$
$Mo_{1,00}V_{0,25}Nb_{0,3}Sb_{0,1}Pd_{0,0004}$

Die erfindungsgemäß verwendeten Katalysatoren können nach den herkömmlichen Verfahren hergestellt werden. Hierzu geht man von einer Aufschlämmung, insbesondere einer wässrigen Lösung, die die einzelnen Ausgangskomponenten der Elemente entsprechend ihrer Anteile enthält, aus.

Die Ausgangsmaterialien der Einzelkomponenten zur Herstellung des erfindungsgemäßen Katalysators sind neben den Oxiden vorzugsweise in Wasser lösliche Substanzen wie Ammoniumsalze, Nitrate, Sulfate, Halogenide, Hydroxide und Salze organischer Säuren, die durch Erwärmung in die entsprechenden Oxide umgewandelt werden können. Zur Vermischung der Komponenten werden wäßrige Lösungen oder Suspensionen der Metallsalze hergestellt und vermischt.

Bei Molybdän empfiehlt es sich aufgrund der kommerziellen Verfügbarkeit als Ausgangsverbindungen die entsprechenden Molybdate, wie z.B. Ammoniummolybdat, einzusetzen.

Als Palladiumverbindungen kommen beispielsweise Palladium(II)-chlorid, Palladium(II)-sulfat, Palladium(II)-tetraminnitrat, Palladium(II)-nitrat sowie Palladium(ll)-acetylacetonat in Frage.

Die erhaltene Reaktionsmischung wird dann 5 Minuten bis 5 Stunden bei 50 bis 100 °C gerührt. Anschließend wird das Wasser entfernt und der verbleibende Katalysator bei einer Temperatur von 50 bis 150°C, insbesondere 80 bis 120°C getrocknet.

Für den Fall, daß der erhaltene Katalysator anschließend noch einem Kalzinierungsprozeß unterworfen wird, empfiehlt es sich den getrockneten und pulverisierten Katalysator bei einer Temperatur im Bereich von 100°C bis 800°C, insbesondere 200 bis 500°C in Gegenwart von Stickstoff, Sauerstoff oder eines sauerstoffhaltigen Gases zu kalzinieren. Die Zeitdauer beträgt 2 bis 24 Stunden.

Der Katalysator kann ohne ein entsprechendes Trägermaterial eingesetzt werden oder mit einem solchen gemischt oder auf ein solches aufgebracht werden. Geeignet sind übliche Trägermaterialien, wie z.B. poröses Siliziumdioxid, geglühtes Siliziumdioxid, Kieselgur, Kieselgel, poröses oder nicht poröses Aluminiumoxid, Titandioxid, Zirkoniumdioxid, Thoriumdioxid, Lanthanoxid, Magnesiumoxid, Calciumoxid, Bariumoxid, Zinnoxid, Cerdioxid, Zinkoxid, Boroxid, Bornitrid, Borcarbid, Borphosphat, Zirkoniumphosphat, Aluminiumsilikat, Siliziumnitrid oder Siliziumcarbid aber auch Glas-, Kohlefaser-, Metalloxid- oder Metallnetze oder entsprechende Monolithe.

Bevorzugte Trägermaterialien haben eine Oberfläche von weniger als 100 $m^2$/kg. Bevorzugte Trägermaterialien sind Siliziumdioxid und Aluminiumoxid mit geringer spezifischer Oberfläche. Der Katalysator kann nach der Formgebung als regelmäßig oder unregelmäßig geformter Trägerkörper oder aber in Pulverform als heterogener Oxidationskatalysator eingesetzt werden.

Die Reaktion kann in der Wirbelschicht oder in einem Festbettreaktor durchgeführt werden. Für den Einsatz in einer Wirbelschicht wird der Katalysator auf eine Korngröße im Bereich von 10 bis 200 µm gemahlen.

Die gasförmige Einspeisung enthält Ethan oder Ethylen und Ethan enthaltende Gemische, welche als reine Gase oder in Mischung mit einem oder mehreren anderen Gasen dem Reaktor zugeführt werden. Als solche zusätzlichen oder Trägergase kommen beispielsweise Stickstoff, Methan, Kohlenmonoxid, Kohlendioxid, Luft und/oder Wasserdampf in Frage. Das molekularen Sauerstoff enthaltende Gas kann Luft oder ein an molekularen Sauerstoff reicheres oder ärmeres Gas als Luft, z.B. Sauerstoff, sein. Der Anteil des Wasserdampfes kann im Bereich von 0 bis 50 Vol% liegen. Höhere Wasserdampfkonzentrationen würden die Aufarbeitung der anfallenden wässrigen Essigsäure aus verfahrenstechnischen Gründen unnötig verteuern. Das Verhältnis von Ethan/Ethylen zu Sauerstoff liegt günstigerweise im Bereich zwischen 1:1 und 10:1, vorzugsweise 2:1 und 8:1. Höhere Sauerstoffgehalte sind bevorzugt, da der erreichbare Ethanumsatz und somit die Ausbetue an Essigsäure höher ist. Bevorzugt ist die Zugabe von Sauerstoff oder des molekularen Sauerstoff enthaltenen Gases in einem Konzentrationsbereich außerhalb der Explosionsgrenzen unter Reaktionsbedingungen, da hierdurch die Durchführung des Verfahrens vereinfacht wird. Allerdings ist es auch möglich das Ethan/Ethylen- zu Sauerstoff-Verhältnis innerhalb der Explosionsgrenzen einzustellen.

Die Reaktion wird bei Temperaturen zwischen 200 und 500°C, bevorzugt 200 bis 400°C durchgeführt. Der Druck kann atmosphärisch oder superatmosphärisch sein, z.B. im Bereich zwischen 1 und 50 bar, bevorzugt 1 bis 30 bar.

Die Reaktion kann in einem Festbett- oder Wirbelschichtreaktor durchgeführt werden. Zweckmäßigerweise wird Ethan zunächst mit den inerten Gasen wie Stickstoff oder Wasserdampf gemischt, bevor Sauerstoff oder das molekularen Sauerstoff enthaltende Gas zugeführt wird. Die vermischten Gase werden bevorzugt in einer Vorheizzone auf die Reaktionstemperatur vorgeheizt, bevor das Gasgemisch mit dem Katalysator in Kontakt gebracht wird. Aus dem Reaktorabgas wird Essigsäure durch Kondensation abgetrennt. Die übrigen Gase werden an den Reaktoreingang zurückgeführt, wo Sauerstoff oder das molekularen Sauerstoff enthaltende Gas sowie Ethan oder das Ethylen und Ethan enthaltende Gasgemisch zudosiert wird.

Bei einem Vergleich der erfindungsgemäßen Katalysatoren mit denen im Stand der Technik bekannten findet man, daß mit den vorliegenden Katalysatoren unter gleichen Reaktionsbedingungen (Reaktionseingangsgas, Druck, Verweilzeit im Reaktor) aber bei wesentlich niedrigeren Temperaturen sogar höhere Essigsäureselektivitäten erreicht werden (Tabelle 1; Bsp. 3 (erfindungsgemäß): Essigsäureselektivität = 77%; Bsp. 13 (EP-0 407 091) Essigsäureselektivität: 60%). Im Vergleich mit der in der US-A-4 250 346 beschriebenen Katalysatorzusammensetzung läßt sich die Selektivität der Reaktion zu Essigsäure mit Hilfe der erfindungsgemäßen Katalysatoren sogar unter niedrigeren Reaktionsdrücken, -temperaturen und Verweilzeiten enorm steigern (vgl. Bsp. 1 (erfindungsgemäß): T = 250°C, p = 7 bar, Verweilzeit = 14s, Essigsäureselektivität: 84%; Bsp. 12 (US-A-4 250 346) T = 280°C, p = 15 bar, Verweilzeit = 30s, Essigsäureselektivität = 32%).

Ebenso lassen sich mit Hilfe der vorliegenden Katalysatoren die Raum-Zeit-Ausbeuten stark erhöhen (Tabelle 1). Raum-Zeit-Ausbeuten kennzeichnen die Menge der produzierten Essigsäure pro Zeit und Katalysatorvolumen. Höhere Raum-Zeit-Ausbeuten sind wünschenswert, da hierdurch die Größe der Reaktoren sowie der Menge des im Kreis geführten Gases verringert werden können.

Bei Verwendung des erfindungsgemäßen Katalysators liegt die Selektivität bei der Oxidation von Ethan und/oder Ethylen zu Essigsäure bei $\geq 60$ Mol%, vorzugsweise $\geq 75$ Mol%, insbesondere $\geq 80$ Mol%, bei einem Ethanumsatz von >4%, vorzugsweise > 5%, insbesondere > 6%, so daß mit dem erfindungsgemäßen Verfahren im Vergleich mit dem Stand der Technik eine Erhöhung der Essigsäureausbeuten, bei gleichzeitiger Verminderung des Anfalls von ungewünschten Nebenprodukten, auf einfache Weise erzielt werden kann.

Beispiele

Die in den Beispielen aufgeführte Katalysatorzusammensetzung ist in relativen Atomverhältnissen angegeben.

Katalysatorpräparation:

Katalysator (I):

Ein Katalysator mit folgender Zusammensetzung wurde hergestellt:
$Mo_{1,00}V_{0,25}Nb_{0,12}Pd_{0,0005}$

Lösung 1:

10,22 g Ammoniummetavanadat in 250 ml Wasser

Lösung 2:

61,75 g Ammoniummolybdat und 0,039 g Palladiumacetat in 200 ml Wasser.

Lösung 3:

27,51 g Nioboxalat in 25 ml Wasser.

Die Lösungen werden separat bei 90°C für 15 Minuten gerührt. Dann wird die dritte Lösung zur ersten hinzugegeben. Die vereinigten Mischungen werden bei 90°C für 15 Minuten gerührt bevor die zweite hinzugegeben wird. Die erhaltene Mischung wird bei 90°C für 15 Minuten gerührt. Anschließend wird das Wasser auf einer heißen Platte entfernt bis eine dicke Paste entsteht. Diese wird bei 120°C über Nacht getrocknet. Der Feststoff wird zerstoßen (Siebfraktion: 0,35 bis 2 mm) und anschließend in statischer Luft bei 400°C für 4 Stunden calciniert. Der Katalysator wird hiernach gesiebt, um eine Siebfraktion zwischen 0,35 und 1 mm zu erhalten.

Katalysator (II):

Ein Katalysator mit folgender Zusammensetzung wurde hergestellt:
$Mo_{1,00}V_{0,25}Nb_{0,12}Pd_{0,0004}$

Die Herstellung erfolgte wie in Katalysatorbeispiel (I) beschrieben, mit der Änderung, daß statt 0,039 g Palladiumacetat 0,031 g eingesetzt wurden.

Katalysator (III):

Ein Katalysator mit folgender Zusammensetzung wurde hergestellt:
$Mo_{1,00}V_{0,36}Nb_{0,03}Sb_{0,01}Ca_{0,01}Pd_{0,0005}$

Lösung 1:

20,0 g Ammoniummolybdat in 100 ml Wasser

Lösung 2:

4,8 g Ammoniummetavanadat in 100 ml Wasser.

Lösung 3:

2,6 g Nioboxalat, 0,48 g Antimonoxalat, 0,34 g Calciumnitrat in 50 ml Wasser.

Lösung 4:

0,013 g Palladiumacetat in 50 ml Aceton.

Die Lösungen 1 bis 3 werden separat bei 70°C für 15 Minuten gerührt. Dann wird die dritte Lösung zur zweiten hinzugegeben. Die vereinigten Mischungen werden bei 70°C für 15 Minuten gerührt bevor diese zur ersten gegeben werden. Hiernach gibt man Lösung 4 hinzu. Die erhaltene Mischung wird bei 70°C für 15 Minuten gerührt. Anschließend wird das Wasser/Aceton-Gemisch rasch verdampft bis eine dicke Paste entsteht. Diese wird bei 120°C über Nacht getrocknet. Der Feststoff wird zerstoßen (Siebfraktion: 0,35 bis 2 mm) und anschließend in statischer Luft bei 300°C für 5 Stunden calciniert. Der Katalysator wird hiernach gesiebt, um eine Siebfraktion zwischen 0,35 und 0,7 mm zu erhalten.

Vergleichsbeispiele

Katalysator (IV):

Zum Vergleich wurde ein Katalysator entsprechend US 4,250,346 mit der Zusammensetzung hergestellt:
$Mo_{1,00}V_{0,25}Nb_{0,12}$

Die Herstellung erfolgte wie in Katalysatorbeispiel (I) beschrieben, mit der Änderung, daß kein Palladiumacetat eingesetzt wurde.

Katalysator (V):

Zum Vergleich wurde ein Katalysator entsprechend EP 0 407 091 mit folgender Zusammensetzung wurde hergestellt:
$Mo_{0,370}Re_{0,248}V_{0,259}Nb_{0,070}Sb_{0,030}Ca_{0,019}$

Lösung 1:

10,0 g Ammoniumperrhenat und 9,7 g Ammoniummolybdat in 50 ml Wasser.

Lösung 2:

4,5 g Ammoniummetavanadat in 50 ml Wasser.

Lösung 3:

6,5 g Nioboxalat, 1,34 g Antimonoxalat, 0,58 g Calciumnitrat in 180 ml Wasser.

Die Lösungen werden separat bei 70°C für 15 Minuten gerührt. Dann wird die dritte Lösung zur zweiten hinzugegeben. Die vereinigten Mischungen werden bei 70°C für 15 Minuten gerührt bevor diese zur ersten gegeben werden. Die erhaltene Mischung wird bei 70°C für 15 Minuten gerührt. Anschließend wird das Wasser auf einer heißen Platte entfernt bis eine dicke Paste entsteht. Diese wird bei 120°C über Nacht getrocknet. Der Feststoff wird zerstoßen (Siebfraktion: 0,35 bis 2 mm) und anschließend in statischer Luft bei 300°C für 5 Stunden calciniert. Der Katalysator wird hiernach gesiebt, um eine Siebfraktion zwischen 0,35 und 1 mm zu erhalten.

Methode zur Katalysatoraustestung

10 ml des Katalysators wurde in einen Stahlreaktor mit 10 mm Innendurchmesser geladen. Der Katalysator wurde unter einem Luftstrom auf 250°C aufgeheizt. Anschließend wurde der Druck mittels eines Vordruckreglers eingestellt. Das gewünschte Ethan: Sauerstoff: Stickstoff-Gemisch wurde mit Wasser in eine Verdampferzone eindosiert, wo Wasser verdampfte und mit den Gasen vermischt wurde. Die Reaktionstemperatur wurde mit einem Thermoelement in der Katalysatorschüttung gemessen. Das Reaktionsgas wurde on-line gaschromatographisch analysiert.

In den Beispielen sind die folgende Begriffe definiert als:

$$Ethanumsatz (\%) =$$

$$100x([CO]/2+[CO_2]/2+[C_2H_4]+[CH_3COOH])/([CO]/_2+[CO_2]/2+[C_2H_4]+[C_2H_6]+ [CH_3COOH])$$

$$Ethylenselektivität (\%) =$$

$$100x([C_2H_4])/([CO]/_2+[CO_2]/2+[C_2H_4]+[CH_3COOH])$$

$$Essigsäureselektiviät (\%) =$$

$$100x([CH_3COOH])/([CO]/_2+[CO_2]/2+[C_2H_4]+[CH_3COOH])$$

worin

[ ] = Konzentrationen in mol% und

$[C_2H_6]$ = Konzentration des nicht umgesetzten Ethans bedeutet.

Die Verweilzeit ist definiert als:

$T_{-}(s)$ = Schüttvolumen des Katalysators (ml) / Volumenstrom des Gases durch den Reaktor bezogen auf die Reaktionsbedingungen (ml/s) Reaktionsdurchführung:

Das Reaktoreingangsgas bestand aus 40 Vol% Ethan, 8 Vol% Sauerstoff, 32 Vol% Stickstoff und 20 Vol% Wasserdampf. Die Reaktionsbedingungen und Ergebnisse sind in nachfolgender Tabelle zusammengefaßt.

Tabelle 1:

| Bsp. | Katalysator | Temperatur (°C) | Druck (bar) | Verweilzeit (s) | Ethan-umsatz (%) | Essigsäure-selektivität (%) | Ethylen-selektivität (%) | Raum-Zeit-Ausbeute [kg/hm³] | CO + CO₂ Selektivität (%) |
|---|---|---|---|---|---|---|---|---|---|
| 1 | (I) | 250 | 7 | 14 | 4 | 84 | 1 | 31 | 15 |
| 2 | (I) | 280 | 7 | 14 | 8 | 76 | 8 | 61 | 16 |
| 3 | (I) | 255 | 15 | 30 | 8 | 77 | 0 | 72 | 23 |
| 4 | (I) | 245 | 15 | 30 | 6 | 82 | 0 | 53 | 18 |
| 5 | (I) | 280 | 15 | 30 | 10 | 77 | 2 | 72 | 21 |
| 6 | (I) | 280 | 28 | 30 | 10 | 80 | 2 | 149 | 18 |
| 7 | (II) | 280 | 7 | 12 | 8 | 73 | 12 | 57 | 15 |
| 8 | (II) | 290 | 7 | 12 | 9 | 70 | 14 | 63 | 16 |
| 9 | (II) | 280 | 15 | 30 | 10 | 79 | 3 | 75 | 18 |
| 10 | (III) | 280 | 15 | 30 | 10 | 86 | 2 | 78 | 12 |
| 11 | (III) | 285 | 15 | 30 | 11 | 86 | 2 | 91 | 12 |
| 12 | (IV) | 280 | 15 | 30 | 9 | 32 | 57 | 28 | 11 |
| 13 | (V) | 280 | 7 | 14 | 4 | 39 | 55 | 4 | 6 |
| 14 | (V) | 280 | 15 | 30 | 4 | 61 | 29 | 28 | 10 |
| 15 | (V) | 300 | 15 | 30 | 9 | 60 | 29 | 28 | 11 |
| 16 | (V) | 300 | 28 | 60 | 9 | 79 | 12 | 92 | 9 |

Im Vergleich zu den Vergleichskatalysatoren (IV) und (V) werden mit den Katalysatoren (I), (II) und (III) bei geringeren Temperaturen und Reaktionsdrucken wesentlich höhere Selektivitäten zu Essigsäure erreicht. Katalysatoren I ($Mo_{1,0}V_{0,25}Nb_{0,12}Pd_{0,0005}$), II ($Mo_{1,0}V_{0,25}Nb_{0,12}Pd_{0,0004}$) und III ($Mo_{1,0}V_{0,36}Nb_{0,03}Sb_{0,01}Ca_{0,01}Pd_{0,0005}$) zeigen im Vergleich zu den Katalysatoren IV ($Mo_{1,0}V_{0,25}Nb_{0,12}$ = US-A-4 250 346) und V ($Mo_{1,0}Re_{0,67}V_{0,70}Nb_{0,19}Sb_{0,08}Ca_{0,05}$ = EP-0 407 091) höhere Raum-Zeit-Ausbeuten.

Vergleichsexperimente zur thermischen Katalysatorstabilität

Um die thermische Stabilität der Katalysatoren zu prüfen wurden die Katalysatoren (I) und (V) in den Reaktor eingebaut und für 100 Stunden betrieben (Reaktionsbedingungen: 280°C, 15 bar, 30 Sekunden Verweilzeit, Zusammensetzung des Reaktionsgases: siehe oben). Nach der Betriebszeit wurde jeweils eine Probe vom Beginn der Katalysatorschüttung entnommen und die Zusammensetzung quantitativ analysiert. In der nachfolgenden Tabelle sind die Zusammensetzungen der gebrauchten und ungebrauchten Katalysatoren gegenübergestellt. .

Tabelle 2:

| Katalysator | Element | Zusammensetzung vor Reaktion (At.-%) | Zusammensetzung nach 100 Stunden Reaktion (At.-%) |
|---|---|---|---|
| (V) | Mo | 38,0 | 44,0 |
| | Re | 23,9 | 13,3 |
| | V | 25,5 | 28,6 |
| | Nb | 7,0 | 8,0 |
| | Sb | 3,7 | 4,1 |
| | Ca | 1,7 | 2,0 |
| | | | |
| (I) | Mo | 72,7 | 72,6 |
| | V | 18,2 | 18,2 |
| | Nb | 8,7 | 8,9 |
| | Pd | 0,4 | 0,4 |

Katalysator (V) hat bereits nach 100 Betriebsstunden 44,4% des eingesetzten Rheniums verloren. Dagegen besitzt der frische und gebrauchte Katalysator (I) die gleiche Zusammensetzungen.

**Patentansprüche**

1. Verfahren zur selektiven Herstellung von Essigsäure aus einer gasförmigen Einspeisung von Ethan oder von Gemischen aus Ethan und Ethylen sowie Sauerstoff bei erhöhter Temperatur, **dadurch gekennzeichnet, daß** die gasförmige Einspeisung mit einem Katalysator zusammengebracht wird, der die Elemente Mo, Pd, X und Y in den Grammatomverhältnissen a:b:c:d in Kombination mit Sauerstoff enthält

$$Mo_aPd_bX_cY_d \qquad\qquad (I)$$

wobei die Symbole X und Y folgende Bedeutung haben

X steht für eines oder mehrere der Elemente ausgewählt aus der Gruppe Cr, Mn, Nb, Ta, Ti, V, Te und W;
Y steht für eines oder mehrere der Elemente ausgewählt aus der Gruppe B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Zr, Hf, Ni, P, Pb, Sb, Si, Sn, Tl und U;
die Indices a, b, c und d stehen für die Grammatomverhältnisse der entsprechenden Elemente, wobei a=1; b>0; c>0; und d=0-2 ist, mit der Maßgabe, daß der Katalysator als X mindestens die Elemente V und Nb enthält und daß Mo und die Elemente für X nicht auf einer Heteropolysäure beruhen.

2. Verfahren nach Anspruch 1, **dadurch gekennzeichnet, daß** X und/oder Y für mehrere Elemente stehen, wobei gegebenenfalls die Indices c und d für verschiedene Elemente unterschiedliche Werte annehmen.

3. Verfahren nach mindestens einem der Ansprüche 1 und 2, **dadurch gekennzeichnet, daß** die Temperatur im Bereich von 200 bis 500°C liegt.

4. Verfahren nach mindestens einem der Ansprüche 1 bis 3, **dadurch gekennzeichnet, daß** der Druck im Reaktor im Bereich von 1 bis 50 bar liegt.

5. Verfahren nach mindestens einem der Ansprüche 1 bis 4, **dadurch gekennzeichnet, daß** b im Bereich von 0,0001 bis 0,5 liegt.

6. Verfahren nach mindestens einem der Ansprüche 1 bis 5, **dadurch gekennzeichnet, daß** dem Reaktor Ethan gemischt mit mindestens einem weiteren Gas zugeführt wird.

7. Verfahren nach Anspruch 6, **dadurch gekennzeichnet, daß** das als weiteres Gas Stickstoff, Sauerstoff, Methan, Kohlenmonoxid, Kohlendioxid, Ethylen und/oder Wasserdampf zugeführt wird.

8. Verfahren nach mindestens einem der Ansprüche 1 bis 7, **dadurch gekennzeichnet, daß** der Katalysator mindestens eine der folgenden Zusammensetzungen in Kornbination mit Sauerstoff enthält:

$Mo_{1,00}V_{0,25}Nb_{0,12}Pd_{0,0005}$
$Mo_{1,00}V_{0,25}Nb_{0,12}Pd_{0,0004}$
$Mo_{1,00}V_{0,25}Nb_{0,12}Pd_{0,0003}$
$Mo_{1,00}V_{0,36}Nb_{0,03}Sb_{0,01}Ca_{0,01}Pd_{0,0005}$
$Mo_{1,00}V_{0,50}Nb_{0,15}Te_{0,2}Pd_{0,0002}$
$Mo_{1,00}V_{0,25}Nb_{0,3}W_{0,2}Pd_{0,0003}$
$Mo_{1,00}V_{0,25}Nb_{0,3}Sb_{0,1}Pd_{0,0004}$

9. Verfahren nach mindestens einem der Ansprüche 1 bis 8, **dadurch gekennzeichnet, daß** der Katalysator mit einem Trägermaterial gemischt oder auf einem Trägermaterial fixiert ist.

10. Verfahren nach mindestens einem der Ansprüche 1 bis 9, **dadurch gekennzeichnet, daß** die Selektivität der Oxidationsreaktion zu Essigsäure $\geq 60\%$, bei einem Ethanumsatz von $\geq 4\%$ beträgt.

11. Katalysator zur selektiven Oxidation von Ethan oder von Gemischen aus Ethan und Ethylen sowie Sauerstoff, enthaltend die Elemente Mo, Pd, X und Y in den Grammatomverhältnissen a:b:c:d in Kombination mit Sauerstoff

$$Mo_aPd_bX_cY_d \tag{I}$$

wobei die Symbole X und Y folgende Bedeutung haben

X steht für eines oder mehrere der Elemente ausgewählt aus der Gruppe: Cr, Mn, Nb, Ta, Ti, V und W;
Y steht für eines oder mehrere der Elemente ausgewählt aus der Gruppe: B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Zr, Hf, Ni, P, Pb, Sb, Si, Sn, Tl und U;
die Indices a, b, c und d stehen für die Grammatomverhältnisse der entsprechenden Elemente, wobei a=1, b>0; c>0 und d=0-2 ist, mit der Maßgabe, daß der Katalysator als X mindestens die Elemente V und Nb enthält und daß Mo und die Elemente für X nicht auf einer Heteropolysäure beruhen.

**Claims**

1. A process for the selective preparation of acetic acid from a gaseous feed comprising ethane or mixtures of ethane and ethylene plus oxygen at elevated temperature, which comprises bringing the gaseous feed into contact with a catalyst comprising the elements Mo, Pd, X and Y in gram atom ratios a:b:c:d in combination with oxygen

$$Mo_aPd_bX_cY_d \qquad (I)$$

where the symbols X and Y have the following meanings:

X is one or more elements selected from the group consisting of Cr, Mn, Nb, Ta, Ti, V, Te and W;
Y is one or more elements selected from the group consisting of B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Zr, Hf, Ni, P, Pb, Sb, Si, Sn, Tl and U;
the indices a, b c and d are the gram atom ratios of the corresponding elements, where
a=1; b>0; c>0; and d=0-2, with the proviso, that the catalyst contains as X at least the elements V and Nb and with the proviso, that Mo and the elements X do not derive from a heteropoly acid.

2. The process as claimed in claim 1, wherein X and/or Y are a plurality of elements, where, if desired, the indices c and d assume different values for different elements.

3. The process as claimed in at least one of claims 1 and 2, wherein the temperature is in the range from 200 to 500°C.

4. The process as claimed in at least one of claims 1 to 3, wherein the pressure in the reactor is in the range from 1 to 50 bar.

5. The process as claimed in at least one of claims 1 to 4, wherein b is in the range from 0.0001 to 0.5.

6. The process as claimed in at least one of claims 1 to 5, wherein ethane mixed with at least one further gas is fed to the reactor.

7. The process as claimed in claim 6, wherein the further gas fed in is nitrogen, oxygen, methane, carbon monoxide, carbon dioxide, ethylene and/or water vapor.

8. The process as claimed in at least one of claims 1 to 7, wherein the catalyst comprises at least one of the following compositions in combinationn with oxygen:

$Mo_{1,00}V_{0,25}Nb_{0,12}Pd_{0,0005}$
$Mo_{1,00}V_{0,25}Nb_{0,12}Pd_{0,0004}$
$Mo_{1,00}V_{0,25}Nb_{0,12}Pd_{0,0003}$
$Mo_{1,00}V_{0,36}Nb_{0,03}Sb_{0,01}Ca_{0,01}Pd_{0,0005}$
$Mo_{1,00}V_{0,50}Nb_{0,15}Te_{0,2}Pd_{0,0002}$
$Mo_{1,00}V_{0,25}Nb_{0,3}W_{0,2}Pd_{0,0003}$
$Mo_{1,00}V_{0,25}Nb_{0,3}Sb_{0,1}Pd_{0,0004}$

9. The process as claimed in at least one of claims 1 to 8, wherein the catalyst is mixed with a support material or is fixed on a support material.

10. The process as claimed in at least one of claims 1 to 9, wherein the selectivity of the oxidation reaction to acetic acid is ≥60%, at an ethane conversion of ≥4%.

11. A catalyst for the selective oxidation of ethane or mixtures of ethane and ethylene plus oxygen, comprising the elements Mo, Pd, X and Y in the gram atom ratios a:b:c:d in combination with oxygen

$$Mo_aPd_bX_cY_d \qquad (I)$$

where the symbols X and Y have the following meanings:

X is one or more elements selected from the group consisting of Cr, Mn, Nb, Ta, Ti, V and W;
Y is one or more elements selected from the group consisting of B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Zr, Hf, Ni, P, Pb, Sb, Si, Sn, Tl and U;

the indices a, b, c and d are the gram atom ratios of the corresponding elements, where a=1; b>0; c>0; and d=0-2, with the proviso, that the catalyst contains as X at least the elements V and Nb and with the proviso, that Mo and the elements X do not derive from a heteropoly acid.

## Revendications

1. Procédé de préparation sélective d'acide acétique à partir d'une charge gazeuse d'éthane ou de mélanges d'éthane et d'éthylène et d'oxygène à haute température, **caractérisé en ce que** la charge gazeuse est mise en contact avec un catalyseur qui contient les éléments Mo, Pd, X et Y dans les proportions en atomes-grammes a:b:c:d en combinaison avec de l'oxygène:

$$Mo_aPd_bX_cY_d \qquad\qquad (I)$$

où les symboles X et Y ont la signification suivante:

  X représente un ou plusieurs éléments choisis dans le groupe constitué par Cr, Mn, Nb, Ta, Ti, V, Te et/ou W;
  Y représente un ou plusieurs éléments choisis dans le groupe constitué par B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Zr, Hf, Ni, P, Pb, Sb, Si, Sn, Tl et U;
  les indices a, b, c et d représentent les proportions en atomes-grammes des éléments correspondants, avec a = 1; b > 0, c > 0 et d = 0 à 2,
  à condition que le catalyseur contienne en tant que X au moins les éléments V et Nb, et que Mo et les éléments de X ne proviennent pas d'un hétéropolyacide.

2. Procédé selon la revendication 1, **caractérisé en ce que** X et/ou Y représentent plusieurs éléments, les indices c et d pour différents éléments prenant éventuellement des valeurs différentes.

3. Procédé selon au moins l'une des revendications 1 et 2, **caractérisé en ce que** la température est comprise entre 200 et 500°C.

4. Procédé selon au moins l'une des revendications 1 à 3, **caractérisé en ce que** la pression dans le réacteur est comprise entre 1 et 50 bars.

5. Procédé selon au moins l'une des revendications 1 à 4, **caractérisé en ce que** b est compris entre 0,0001 et 0,5.

6. Procédé selon au moins l'une des revendications 1 à 5, **caractérisé en ce que** l'on introduit dans le réacteur de l'éthane mélangé avec au moins un autre gaz.

7. Procédé selon la revendication 6, **caractérisé en ce que** l'on introduit comme autre gaz de l'azote, de l'oxygène, du méthane, du monoxyde de carbone, du dioxyde de carbone, de l'éthylène et/ou de la vapeur d'eau.

8. Procédé selon au moins l'une des revendications 1 à 7, **caractérisé en ce que** le catalyseur contient au moins l'une des compositions suivantes en combinaison avec de l'oxygène:

  $Mo_{1,00}V_{0,25}Nb_{0,12}Pd_{0,0005}$
  $Mo_{1,00}V_{0,25}Nb_{0,12}Pd_{0,0004}$
  $Mo_{1,00}V_{0,25}Nb_{0,12}Pd_{0,0003}$
  $Mo_{1,00}V_{0,36}Nb_{0,03}Sb_{0,01}Ca_{0,01}Pd_{0,0005}$
  $Mo_{1,00}V_{0,50}Nb_{0,15}Te_{0,2}Pd_{0,0002}$
  $Mo_{1,00}V_{0,25}Nb_{0,3}W_{0,2}Pd_{0,0003}$
  $Mo_{1,00}V_{0,25}Nb_{0,3}Sb_{0,1}Pd_{0,0004}.$

9. Procédé selon au moins l'une des revendications 1 à 8, **caractérisé en ce que** le catalyseur est mélangé avec un support ou est fixé sur un support.

10. Procédé selon au moins l'une des revendications 1 à 9, **caractérisé en ce que** la sélectivité de la réaction d'oxydation pour l'acide acétique est égale ou supérieure à 60 %, avec un taux de conversion de l'éthane égal ou supérieur à 4 %.

11. Catalyseur pour l'oxydation sélective d'éthane ou de mélanges d'éthane et d'éthylène et d'oxygène, contenant les éléments Mo, Pd, X et Y dans les proportions en atomes-grammes a:b:c:d en combinaison avec de l'oxygène:

$$Mo_a Pd_b X_c Y_d \qquad\qquad (I)$$

où les symboles X et Y ont la signification suivante:

X représente un ou plusieurs éléments choisis dans le groupe constitué par Cr, Mn, Nb, Ta, Ti, V et W;

Y représente un ou plusieurs éléments choisis dans le groupe constitué par B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Zr, Hf, Ni, P, Pb, Sb, Si, Sn, Tl et U;

les indices a, b, c et d représentent les proportions en atomes-grammes des éléments correspondants, avec a = 1; b > 0, c > 0 et d = 0 à 2,

à condition que le catalyseur contienne en tant que X au moins les éléments V et Nb, et que Mo et les éléments de X ne proviennent pas d'un hétéropolyacide.